(19) Europäisches Patentamt / European Patent Office / Office européen des brevets

(11) **EP 2 040 719 B1**

(12) **FASCICULE DE BREVET EUROPEEN**

(45) Date de publication et mention de la délivrance du brevet:
**29.02.2012 Bulletin 2012/09**

(21) Numéro de dépôt: **07731481.3**

(22) Date de dépôt: **18.05.2007**

(51) Int Cl.:
*A61K 35/12* (2006.01)      *A61P 35/00* (2006.01)
*A61K 48/00* (2006.01)      *C12N 5/074* (2010.01)

(86) Numéro de dépôt international:
**PCT/FR2007/000847**

(87) Numéro de publication internationale:
**WO 2007/135284 (29.11.2007 Gazette 2007/48)**

(54) **UTILISATION DE CELLULES DERIVEES DU TISSU ADIPEUX POUR LA PREPARATION D'UN MEDICAMENT ANTI-TUMORAL**

VERWENDUNG VON ZELLEN AUS FETTGEWEBE ZUR HERSTELLUNG EINES ANTITUMORALEN MEDIKAMENTS

USE OF CELLS DERIVED FROM ADIPOSE TISSUE FOR THE PREPARATION OF AN ANTI-TUMOUR MEDICAMENT

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **18.05.2006 FR 0604443**

(43) Date de publication de la demande:
**01.04.2009 Bulletin 2009/14**

(73) Titulaires:
• **Centre National de la Recherche Scientifique**
  **75016 Paris (FR)**
• **UNIVERSITE PAUL SABATIER TOULOUSE III**
  **31062 Toulouse Cédex 4 (FR)**
• **Institut National De La Sante Et De La Recherche Medicale**
  **75013 Paris (FR)**

(72) Inventeurs:
• **CORDELIER, Pierre**
  **31000 Toulouse (FR)**
• **BUSCAIL, Louis**
  **31200 Toulouse (FR)**
• **CASTEILLA, Louis**
  **31750 Escalquens (FR)**
• **COUSIN-DELARUE, Béatrice**
  **31560 Nailloux (FR)**
• **PENICAUD, Luc**
  **31400 Toulouse (FR)**
• **PERON, Jean-Marie**
  **31100 Toulouse (FR)**

(74) Mandataire: **Leblois-Préhaud, Hélène Marthe Georgette et al**
**Cabinet Orès**
**36, rue de St Petersbourg**
**75008 Paris (FR)**

(56) Documents cités:
**EP-A2- 1 077 254       WO-A-02/055678**
**WO-A-2005/025584       WO-A2-01/62901**
**WO-A2-03/039481        FR-A- 2 859 381**
**FR-A1- 2 819 265       US-A1- 2003 124 721**
**US-B1- 6 555 374**

EP 2 040 719 B1

**Description**

La présente Invention est relative à l'utilisation de cellules dérivées du tissu adipeux pour la préparation d'un médicament à action anti-tumorale.

Le traitement efficace des cancers reste l'un des défis majeurs de la médecine d'aujourd'hui.

L'efficacité des thérapeutiques conventionnelles chirurgicales ou à visée cytolytique (chimiothérapie et radiothérapie) reste très limitée dans de nombreux cancers. En effet, certains cancers de fréquence élevée (tractus gastro-intestinal, en particulier), sont toujours difficiles à traiter compte tenu de leur aspect massif qui s'oppose à la diffusion des agents thérapeutiques. Leur traitement nécessite donc en premier lieu l'exérèse chirurgicale, suivie d'une chimiothérapie éventuellement associée à une radiothérapie. Toutefois, dans les cas où la chirurgie n'est pas ou plus possible, une chimiothérapie alternative et efficace sans être toxique est nécessaire. Toutefois, les produits disponibles présentent une efficacité limitée ou une toxicité inacceptable. Il existe donc un besoin de nouveaux traitements anti-cancéreux, afin de disposer d'une panoplie suffisamment étendue de traitements, pour augmenter ainsi les chances de guérison.

Dans le cas des cancers du tractus gastro-intestinal (oesophage, estomac, intestin grêle, gros intestin, colon) et de ses glandes annexes (foie, vésicule biliaire, pancréas), par exemple, le manque de traitements curatifs est avéré, lorsque l'exérèse chirurgicale n'est pas ou plus possible. En effet, les traitements existants, qui sont essentiellement basés sur la chimiothérapie (5-fluorouracile ou 5-FU, gemcitabine...), et la radiothérapie, ont un faible impact sur la survie et sont surtout utilisés à des fins palliatives, notamment dans le cas des cancers colorectaux, gastriques et pancréatiques (pour revue, DIAZ-RUBIO, The Oncologist, 2004, 9, 282-294).

Outre l'absence de traitements efficaces, la toxicité des agents chimiothérapeutiques habituellement utilisés (fluoro-uracile, acide folinique, dérivés du platine, tel qu'oxaliplatine, mitomycine C, par exemple) et les effets secondaires associés à ces traitements représentent un autre inconvénient majeur.

De nouveaux anticancéreux ont été testés, notamment dans les cancers du tractus gastro-intestinal tels que le pemetrexed (antifolate : agent cytotoxique), des vaccins, des inhibiteurs de kinase (bryostatin, UCN-01, flavopiridol, CI-1040), des inhibiteurs de l'EGFR (cetuximab, gefitinib, GW572016, CI-1033, erlotinib) et des inhibiteurs du VEGF (bevacizumab, PTK787/ZK 222584, angiozyme, ZD6474).

Par exemple :

- le cetuximab est un anticorps monoclonal chimérique qui se lie spécifiquement au domaine extra-cellulaire du récepteur du facteur de croissance épidermique humaine (EGFR) et inhibe la prolifération de cellules tumorales exprimant l'EGFR et induit une apoptose.
- le bevacizumab est un anticorps monoclonal de type IgG1 qui se lie au VEGF (Vascular Endothelial Growth Factor) et inhibe de ce fait la liaison du VEGF à ses récepteurs, Flt-1 (VEGFR-1) et KDR (VEGFR-2), situés à la surface des cellules endothéliales aussi bien *in vitro* qu'*in vivo.* Il est constitué d'une partie constante d'origine humaine et d'une partie variable d'origine murine.

Les résultats obtenus avec ces nouveaux produits seuls ou en association ne montrent pas d'amélioration par rapport aux traitements antérieurs, en particulier dans le traitement des cancers du tractus digestif, tant du point de vue de l'efficacité que de la toxicité.

Dans le cas du cancer du pancréas, qui représente la cinquième cause de décès par cancer dans les pays occidentaux, le médecin est encore plus démuni ; outre une agressivité et une révélation clinique souvent tardive, son pronostic est très mauvais (survie à 5 ans inférieure à 3,5 %) ; de surcroît, l'exérèse chirurgicale n'est possible que dans 10 à 15 % des cas. La radiothérapie ou la chimiothérapie n'ont que peu d'effet sur la survie des patients dont la tumeur n'a pas été réséquée (médiane de survie 5-6 mois) (Safioleas MC et al., Hepatogastroenterology, 2004, 51 (57) : 862-868 ; Jemal A. et al., CA Cancer J Clin, 2002, 52 (1) : 23-47 ; Rosewicz S., et al., Lancet, 1997, 349 (9050) : 485-489., Jafari M. et al., Surg Oncol Clin N Am, 2004, 13 (4) : 751-760, xi ; Kullke MH et al., Curr Treat Options Oncol, 2002, 3 (6) : 449-457). Les cancers du pancréas non-résécables, notamment le cancer de la tête du pancréas, font l'objet de dérivations palliatives, de la mise en place d'endoprothèses biliaires ou duodénales, voire d'alcoolisation antalgique du plexus coeliaque. La survie des patients demeure très faible :

- En cas de cancer localement avancé non-métastatique et non résécable ou de cancer métastatique, le traitement de référence actuel est la gemcitabine, avec laquelle on observe une amélioration des signes cliniques (douleur, troubles du transit) alors que le pronostic vital reste faible (7 à 10 mois) (Safioleas MC et al., Hepatogastroenterology, 2004, 51 (57): 862-868 ; Jemal A. et al., CA Cancer J Clin, 2002, 52 (1): 23-47 ; Rosewicz S., et al., Lancet, 1997, 349 (9050) : 485-489., Jafari M. et al., Surg Oncol Clin NAm, 2004, 13 (4) : 751-760, xi ; Kullke MH et al., Curr Treat Options Oncol, 2002, 3 (6) : 449-457).
- Une des voies de recherche pour améliorer le pronostic du cancer du pancréas est de disposer d'un traitement efficace applicable notamment aux tumeurs non résécables dans le but de réduire la progression de la maladie.

Des protocoles d'investigations cliniques utilisant la thérapie génique ou la thérapie cellulaire ont ainsi été mis en place :

- Par exemple, MULVIHILL et al. (Gene Therapy, 2001, 8, 308-315) ont conduit des essais cliniques de phase I, au cours desquels ils ont procédé à l'injection intra-tumorale, à l'aide d'un dispositif de tomographie assistée par ordinateur (CT pour « *computed tomography* »), chez des patients atteints d'un carcinome de pancréas non-résécable, de l'adénovirus ONYX-015 (*dl1520*), un adénovirus délété au niveau du gène *E1B-55kD* qui se réplique préférentiellement dans les cellules tumorales dépourvues de protéine p53 et tue celles-ci. L'injection d'adénovirus est bien tolérée mais aucune réponse objective n'est démontrée. Les Auteurs soulignent que la réplication virale intra-tumorale n'est pas assez importante et que l'efficacité des adénovirus pourrait être optimisée en multipliant les injections d'ONYX-015 ou bien en les associant avec un traitement chimiothérapeutique.
- Plus récemment, HECHT et al. (Clinical Cancer Research, 2003, 9, 555-561) ont modifié ce protocole en proposant de remplacer le dispositif de CT, trop encombrant et susceptible de provoquer des complications sérieuses telles que des infections ou des perforations, par l'endoscopie à ultrasons. Cependant, les essais en phases I/II conduits par cette équipe n'ont pas révélé d'amélioration de l'efficacité du traitement par ONYX-015. En outre, des cas de perforations duodénales, dus à l'endoscope ont été observés.
- D'autres adénovirus ont été testés pour traiter des tumeurs digestives. C'est ainsi que l'équipe de SANGRO et al. (Journal of Clinical Oncology, 2004, 22, 8, 1389-1397) a décrit l'injection intra-tumorale d'un adénovirus exprimant l'interleukine 12 (IL-12), dénommé Ad.IL-12, chez des patients atteints de cancers pancréatiques, colorectaux ou hépatiques. Ces essais de phase 1 n'ont révélé qu'une activité anti-tumorale modérée. Toujours en orientant ses recherches sur l'expression intra-tumorale d'IL-12 par l'adénovirus Ad.IL-12 (également dénommé AFIL-12), la même équipe a plus récemment testé l'injection intra-tumorale de cellules dendritiques transfectées par l'adénovirus AFIL-12 (MAZZOLINI et al., Journal of Clinical Oncology, 2005, 23, 5, 999-1010). Cependant, des essais cliniques supplémentaires sont nécessaires pour évaluer la réelle efficacité de ce traitement.
- Enfin, l'administration intra-tumorale d'un vecteur adénoviral codant pour le TNF-$\alpha$ associé à une radio-chimiothérapie par 5-FU a également été expérimentée. Cet essai fait état d'une activité anti-tumorale aux doses maximales de vecteur adénoviral-TNF-$\alpha$ (Senzer N. et al., J Clin Oncol, 2004, 22 (4) : 592-601). Toutefois, l'injection d'un vecteur adénoviral s'accompagne généralement d'évènements indésirables tels que la fièvre, des nausées, une lymphopénie etc..

Outre les stratégies faisant appel aux adénovirus, des thérapies utilisant l'approche des « gènes-suicide » (en anglais : GDEPT pour « *gene-derived enzyme prodrug therapy* »), c'est-à-dire associant l'administration d'une pro-drogue et d'un gène dont le produit de traduction métabolise ladite pro-drogue en dérivé(s) actif(s) toxique(s) pour la cellule tumorale, ont également été développées et testées contre des cancers du tractus gastro-intestinal tel que le cancer du pancréas. L'article de GÜNZBURG et SALMONS (Acta Biochimica Polonica, 2005, 52, 3, 601-607) fait le point sur cette approche associée à la thérapie cellulaire dans le cadre du cancer du pancréas. Les Auteurs de cet article soulignent le besoin de la mise au point de nouvelles stratégies plus efficaces de traitement du cancer, que les traitements existants. C'est ainsi que les Auteurs de cet article ont proposé d'administrer, dans des cancers du pancréas non-opérables, des microcapsules de sulfate de cellulose contenant des cellules HEK 293 recombinantes exprimant le cytochrome P450 2B1, en association avec l'ifosfamide et ce pour diminuer les doses efficaces d'ifosfamide, habituellement très toxique, en raison d'une demi-vie très courte de la forme plasmatique active. L'administration des microcapsules est effectuée soit directement dans la tumeur, soit dans la circulation vasculaire alimentant cette tumeur, et permet de concentrer le cytochrome P450 au niveau de ladite tumeur et donc de cibler l'action des métabolites de l'ifosfamide. (LÖHR et al., The Lancet, 2001, 357, 1591-1592). Les essais cliniques conduits jusqu'à présent chez l'homme ont montré un doublement de la médiane de survie (LÖHR et al., 2001) et un taux de survie multiplié par 3 (LÖHR et al., 2001 ; GÜNZBURG et SALMONS, 2005). Cependant, les résultats obtenus ne sont pas satisfaisants et ne permettent pas d'augmenter la survie des patients de manière significative (médiane de survie de 10 mois et taux de survie après un an de 35,7 %). Plus récemment, cette technique a été étendue à d'autres types de cancers mais cela reste, pour le moment, limité aux modèles animaux. Ainsi, SAMEL et al. (Cancer Gene Therapy, 2006, 13, 65-73) appliquent la « chimiothérapie ciblée » à des modèles murins et montrent que l'injection desdites microcapsules associée à une administration d'ifosfamide chez des souris développant un cancer colorectal humain associé à une carcinose péritonéale peut conduire à une rémission complète de la tumeur péritonéale.

La thérapie cellulaire mettant en oeuvre des cellules microencapsulées présente l'inconvénient majeur d'utiliser une lignée cellulaire humaine tumorigène (lignée HEK 293).

Il existe donc un besoin de développer de nouvelles stratégies thérapeutiques anti-cancéreuses, adaptées à tous les cancers et notamment adaptées au traitement des tumeurs solides, telles que les tumeurs du tractus gastro-intestinal.

Les Inventeurs se sont donc donné pour but de proposer un nouveau type de thérapie anticancéreuse, qui, associée à d'autres thérapeutiques, est efficace et moins toxique que les traitements actuellement proposés ; cette thérapie est particulièrement bien adaptée aux cancers du tractus gastro-intestinal, tel que des cancers du pancréas non-opérables.

La présente invention a pour objet l'utilisation de cellules isolées du tissu adipeux blanc extra-médullaire, sélectionnées dans le groupe constitué par la fraction stroma-vasculaire et une sous-population de ladite fraction stroma-vasculaire constituée par des cellules adhérentes, pour la préparation d'un médicament anti-tumoral ayant pour effet le ralentissement ou l'inhibition de la croissance tumorale et l'induction de la mort des cellules cancéreuses.

Le tissu adipeux existe sous différentes formes chez les mammifères : le tissu adipeux blanc extra-médullaire qui représente le principal organe de réserve de l'organisme, le tissu adipeux blanc médullaire dont le rôle exact n'est pas connu et le tissu adipeux brun thermogénique.

Du fait de son potentiel d'expansion important qui persiste tout au long de la vie de l'individu, le tissu adipeux blanc de l'adulte constitue une source de cellules abondantes et faciles à obtenir.

Ce tissu adipeux blanc est constitué par deux fractions cellulaires :

- une fraction adipocytaire qui représente 30% à 60% des cellules du tissu adipeux et est caractérisée par l'accumulation de triglycérides (fraction de cellules flottantes). Cette fraction est composée en grande majorité (99 %) d'adipocytes différenciés et de quelques macrophages contaminants, riches en gouttelettes lipidiques, et
- une fraction non-adipocytaire, appelée fraction stroma-vasculaire (FSV ou en anglais SVF pour « *stroma-vascular fraction* »).

Ces deux fractions cellulaires peuvent être séparées par leur différence de densité, selon des procédés tels que ceux décrits par BJÖRNTORP et al. (J. Lipid. Res., 1978, 19, 316-24).

La fraction stroma-vasculaire, classiquement utilisée pour étudier la différenciation des préadipocytes en adipocytes matures, est une fraction hétérogène comprenant différentes sous-populations de cellules (PLANAT-BERNARD V. et al., Circulation, 2004, 109, 656-663 ; ZUK PA. et al., Mol. Biol. Cell, 2002, 13, 4279-95 ; ERICKSON GR. et al., Biochem. Biophys. Res. Commun., 2002, 290, 763-9 ; COUSIN B. et al., biochem. Biophys. Res. Commun., 2003, 301, 1016-22 ; SAFFORD KM. et al., Biochem. Biophys. Res. Commun., 2002, 294, 371-9 ; Demande Internationale WO 02/055678 et Demande Américaine US 2003/0082152).

Plus précisément, les Inventeurs et d'autres équipes ont précédemment montré qu'il est possible d'induire la différenciation des cellules indifférenciées de la FSV en différents types de cellules différenciées. Les cellules FSV sont en effet capables de se différencier en cellules exprimant des marqueurs spécifiques :

- des cellules hématopoïétiques (Demande Internationale WO 02/055678, Demande Européenne EP 1 077 254 ; Brevet US 6,555,374, Demande Internationale WO 01/62901),
- des cellules musculaires lisses ou squelettiques (Demande Internationale WO 02/055678, Demande Européenne EP 1 077 254 ; Brevet US 6,555,374),
- des cellules musculaires cardiaques (Demande Internationale WO 02/055678),
- des cellules endothéliales, hépatiques, neuronales ou astrogliales (Demande Internationale WO 01/62901),
- des cellules pancréatiques (Demande US 2003/0124721) ou
- des cellules stromales intra-oculaires (Demande Internationale WO 03/039481).

De plus, une sous-population de cellules homogènes de la FSV exprimant les antigènes de surface CD13 et HLA ABC est capable de se différencier en cellules endothéliales (Demande Internationale WO 2005/025584).

Selon l'ensemble de ces publications, les cellules différenciées obtenues à partir des cellules FSV trouvent applications dans la réparation tissulaire, la reconstitution de lignées cellulaires et l'amélioration des fonctions de certains tissus. Ainsi, ces cellules différenciées peuvent être utilisées, selon les cas :

- pour reconstituer les lignées hématopoïétiques dans le cadre du traitement des maladies dans lesquelles on observe une déplétion médullaire, par exemple pour repeupler la moelle osseuse de patients immunodéprimés suite à un traitement anticancéreux tel qu'un traitement par irradiation ;
- pour réparer ou reconstruire le tissu nerveux, par exemple dans le cadre du traitement de pathologies cérébrales, tels qu'un accident vasculaire cérébral, la maladie d'Alzheimer ou la maladie de Parkinson ;
- pour reconstituer le tissus hépatique, par exemple dans le cadre du traitement d'une dégénérescence progressive du foie ;
- pour reconstituer le tissu musculaire cardiaque ou squelettique, notamment dans le cadre du traitement de myopathies, des cardiomyopathies et des pathologies liées à une dégénérescence musculaire (infarctus du myocarde) ;

- pour améliorer les fonctions du tissu pancréatique observé dans certains désordres endocrines du pancréas ;
- pour la réparation ou la reconstruction du tissu intra-oculaire dans le cadre du traitement de lésions du tissu cornéen ou du tissu conjonctif, par exemple après la résection d'une tumeur ; et
- pour reconstruire totalement ou partiellement un réseau vasculaire fonctionnel, notamment dans le cadre du traitement de l'ischémie.

Pour la reconstitution des lignées hématopoïétiques et la réparation du tissu nerveux ou hépatique la Demande Internationale WO 01/62901 préconise également d'injecter les cellules stromales indifférenciées, et non les cellules différenciées.

De façon surprenante, les Inventeurs ont maintenant mis en évidence l'activité anti-tumorale des cellules du tissu adipeux blanc extra-médullaire sélectionnées dans le groupe constitué par la fraction stroma-vasculaire et une sous-population de cellules isolées de ladite fraction stroma-vasculaire, constituée par les cellules qui, après mise en culture primaire de ladite FSV, adhèrent au support de culture.

Cette sous-population de cellules adhérentes sera par la suite indifféremment désignée par les termes « sous-population de cellules adhérentes », « cellules adhérentes », ou « cellules adhérentes de la FSV ».

Dans la suite « ensemble des cellules de la FSV » ou « FSV » ont la même signification.

Au sens de la présente invention, on entend par FSV, la fraction stroma-vasculaire comprenant l'ensemble des cellules la constituant. Dans la suite, et sauf précision contraire, l'expression « cellules de la fraction stroma-vasculaire » inclut aussi bien la fraction stroma-vasculaire complète que la sous-population de cellules adhérentes.

La sous-population de cellules adhérentes représente environ 50 à 60 % de la population cellulaire totale de la FSV.

De manière surprenante, aussi bien la FSV que ladite sous-population de cellules adhérentes ralentissent de manière significative la progression tumorale. Cet effet est observé non seulement lorsque la fraction stroma-vasculaire ou lesdites cellules adhérentes sont injectées localement au niveau intra-tumoral, mais également lorsqu'elles sont administrées de façon systémique, par exemple par voie parentérale et plus précisément par voie intra-veineuse.

Également de manière surprenante, la FSV peut être utilisée pour la préparation dudit médicament anti-tumoral, avec ou sans expansion, avec ou sans traitement physiologique ou pharmacologique et avec ou sans modification par une quelconque manipulation du profil d'expression génique ou protéique ; les cellules adhérentes peuvent être utilisées pour la préparation dudit médicament anti-tumoral, avec ou sans traitement physiologique ou pharmacologique et avec ou sans modification par une quelconque manipulation du profil d'expression génique ou protéique.

Également de manière surprenante, outre l'effet anti-tumoral direct des cellules de la fraction stroma-vasculaire, lesdites cellules exercent également un effet anti-tumoral de façon indirecte : en effet, un milieu conditionné par la sous-population de cellules adhérentes inhibe la viabilité des cellules cancéreuses. Cet effet est en outre amplifié lorsque ladite sous-population de cellules adhérentes est préalablement mise en présence de cellules cancéreuses, de préférence des cellules issues du cancer à traiter.

Les Inventeurs ont en outre montré que, de manière étonnante, cet effet anti-tumoral est associé à une induction *in vitro* et *in vive* de la mort cellulaire des cellules cancéreuses.

De manière remarquable, il n'est pas nécessaire d'induire la différenciation des cellules FSV ou de les faire surexprimer un facteur particulier pour qu'elles exercent leur effet anti-tumoral. L'effet est observé lorsqu'on met en oeuvre lesdites cellules FSV après leur purification ou bien après une culture primaire et sélection des cellules adhérentes.

Selon une disposition avantageuse de ladite utilisation, ledit médicament anti-tumoral est constitué par lesdites cellules isolées, avant ou après mise en culture.

Conformément à l'invention, ladite sous-population de cellules adhérentes est susceptible d'être obtenue par un procédé comprenant :

- l'obtention de la fraction stroma-vasculaire à partir du tissu adipeux blanc extra-médullaire ;
- la purification de ladite fraction stroma-vasculaire ;
- l'isolement des cellules de ladite sous-population à partir de ladite fraction purifiée par mise en culture primaire dans un milieu liquide convenable, sélection des cellules adhérentes sur un support de culture (plastique...) par élimination des cellules non-adhérentes, récupération des cellules après confluence, dans un milieu convenable, centrifugation et récupération du culot.

Un exemple du procédé d'obtention de ces cellules est décrit dans l'article de BJÖRNTORP et al. (cité plus haut).

Selon une modalité avantageuse de cette disposition, lesdites cellules sont associées à un véhicule pharmaceutiquement acceptable.

Conformément à l'invention, lesdites cellules peuvent de surcroît être génétiquement modifiées :

- Elles peuvent comprendre au moins une mutation d'un gène autologue.

- Elles peuvent contenir au moins une copie d'un gène hétérologue.

Selon un autre mode de réalisation avantageux, lesdites cellules comprennent un gène hétérologue, dont le produit de traduction est une protéine d'intérêt thérapeutique, telle qu'une enzyme capable de métaboliser une pro-drogue en composé(s) actif(s) toxique(s) pour la cellule tumorale.

Lesdites cellules génétiquement modifiées sont de préférence d'origine humaine.

Selon une autre disposition avantageuse de ladite utilisation, ledit médicament anti-tumoral est constitué par le surnageant de culture de ladite souspopulation de cellules adhérentes.

Ledit surnageant est un surnageant de culture primaire ou un surnageant de culture obtenu après un ou plusieurs passages (culture secondaire ou cultures ultérieures).

Selon une modalité avantageuse de cette disposition, ledit surnageant est obtenu à partir d'une co-culture de la sous-population de cellules adhérentes telle que définie plus haut avec des cellules cancéreuses ou avec des cellules d'une lignée cellulaire cancéreuse.

Le tissu adipeux extra-médullaire est d'origine animale ou d'origine humaine ; de préférence, aussi bien les cellules de la fraction stroma-vasculaire du tissu adipeux extra-médullaire que les cellules cancéreuses sont celles du patient à traiter et ont été préalablement prélevées chez ledit patient à traiter.

Ledit surnageant est un surnageant de culture primaire ou un surnageant de culture obtenu après un ou plusieurs passages (culture secondaire ou cultures ultérieures).

Selon un autre mode de réalisation avantageux de ladite utilisation, ledit cancer est un cancer solide ou un cancer liquide.

On entend par cancer solide, tout cancer touchant les organes tels que le foie, le pancréas, les poumons, les reins etc., pour lesquels une tumeur se développe localement puis se disperse par la circulation sanguine ou lymphatique et forme des métastases. Cancer solide s'oppose aux cancers liquides qui concernent les cancers du sang ou du système lymphatique.

Selon une disposition préférée de ce mode de réalisation, ledit cancer est un cancer solide du tractus gastro-intestinal, tel que cancer du pancréas, cancer gastrique, cancer colo-rectal.

On entend par cancer du tractus gastro-intestinal les tumeurs et /ou cancers de l'oesophage, de l'estomac, de l'intestin grêle, du gros intestin, ou du colon ainsi que les cancers ou tumeurs des glandes annexes du tractus gastro-intestinal telles que le foie, la vésicule biliaire, le canal cholédoque et le pancréas.

De manière préférée, le médicament anti-tumoral tel que défini ci-dessus est particulièrement bien adapté au traitement du cancer du pancréas, et de manière encore plus préférée au traitement du cancer du pancréas non-opérable.

Dans un tel cas, lorsque l'on utilise une co-culture de la sous-population de cellules adhérentes et d'une lignée cellulaire cancéreuse, cette dernière est avantageusement la lignée de cellules pancréatique Capan-1 (ATCC HTB-79).

Le médicament anti-tumoral selon l'invention est utilisé préférentiellement par voie intra-tumorale, mais il peut aussi être administré par toutes autres voies, éventuellement par voies multiples, notamment par voies intraveineuse, intra-péritonéale, topique, transdermique, sous-cutanée, intra-artérielle, pulmonaire, naso-pharyngée ou orale, en solution, en suspension aqueuse ou en poudre ou sous toute autre forme pharmaceutiquement acceptable.

Les doses efficaces seront déterminées en fonction de l'âge, de l'état de santé, du poids du patient et du type de cancer à traiter.

Conformément à l'invention, l'utilisation du médicament anti-tumoral, tel que défini ci-dessus, peut être combinée avec d'autres thérapies, notamment la chirurgie, la radiothérapie, la chimiothérapie, l'immunothérapie et les thérapies différenciantes.

L'effet anti-tumoral observé, caractérisé par le ralentissement ou l'inhibition de la croissance tumorale et l'induction de la mort des cellules cancéreuses (par exemple apoptose) est essentiellement dû aux cellules de la sous-population cellulaire telle que définie plus haut, c'est-à-dire aux cellules présentes dans la fraction stroma-vasculaire et sélectionnées sur la base de leur adhérence sur support solide (support de culture) lors d'une culture primaire des cellules FSV ; toutefois aussi bien l'ensemble de la fraction stroma-vasculaire que les cellules de ladite sous-population peuvent être mises en oeuvre dans l'invention.

Le médicament anti-tumoral tel que défini ci-dessus (sous-population de cellules adhérentes FSV, ou surnageant de culture des cellules de ladite sous-population) est particulièrement avantageux pour les raisons suivantes :

- le prélèvement d'échantillons de tissu adipeux est facile à réaliser, par exemple par liposuccion ou par biopsie sous anesthésie locale ;
- compte tenu de son abondance, des stocks peuvent être aisément constitués ; en outre, le tissu prélevé peut être rapidement régénéré chez le sujet prélevé ;
- ces propriétés rendent les cellules dérivées du tissu adipeux particulièrement adaptées à une greffe homologue (par exemple greffe autologue) ou hétérologue ;
- le prélèvement de tissu adipeux et son utilisation ultérieure à des fins thérapeutique ne devraient théoriquement rencontrer aucun obstacle d'ordre éthique puisque le prélèvement est peu invasif et beaucoup de prélèvements de tissus adipeux sont actuellement détruits ; en outre, il convient de noter que le temps d'hospitalisation nécessaire audit prélèvement est réduit (notamment il n'est pas besoin de recourir à une cytaphérèse ou à une anesthésie générale).

- il est possible de maintenir et multiplier, voire immortaliser ces cellules *in vitro* dans un milieu défini ; par ailleurs, ces cellules sont transfectables et peuvent être utilisées pour exprimer un gène hétérologue, d'autant plus qu'elles possèdent un fort pouvoir sécréteur. Il est donc possible de les utiliser pour exprimer une protéine thérapeutique, par exemple une enzyme capable de convertir une pro-drogue en drogue active.

La présente invention a également pour objet l'utilisation d'un agent anti-tumoral tel que défini ci-dessus, à savoir sélectionné dans le groupe constitué par des cellules du tissu adipeux blanc extra-médullaire telles que définies ci-dessus, le surnageant de culture desdites cellules ou le surnageant d'une co-culture desdites cellules avec des cellules cancéreuses ou des cellules d'une lignée cellulaire cancéreuse convenable, comme adjuvant en thérapie anticancéreuse.

De préférence, ledit surnageant est un surnageant de culture primaire ou un surnageant de co-culture primaire. Il peut également s'agir d'un surnageant de culture ou de co-culture obtenu après un ou plusieurs passages.

La présente invention a également pour objet l'utilisation d'un agent anti-tumoral tel que défini ci-dessus, à savoir sélectionné dans le groupe constitué par des cellules du tissu adipeux blanc extra-médullaire telle que définie ci-dessus, le surnageant de culture desdites cellules ou le surnageant d'une co-culture desdites cellules avec des cellules cancéreuses ou des cellules d'une lignée cellulaire cancéreuse convenable, pour cribler *in vitro* d'autres médicaments anti-tumoraux, notamment aptes à agir en synergie avec lesdites cellules ou surnageant desdites cellules.

De préférence, ledit surnageant est un surnageant de culture primaire ou un surnageant de co-culture primaire. Il peut également s'agir d'un surnageant de culture ou de co-culture obtenu après un ou plusieurs passages.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

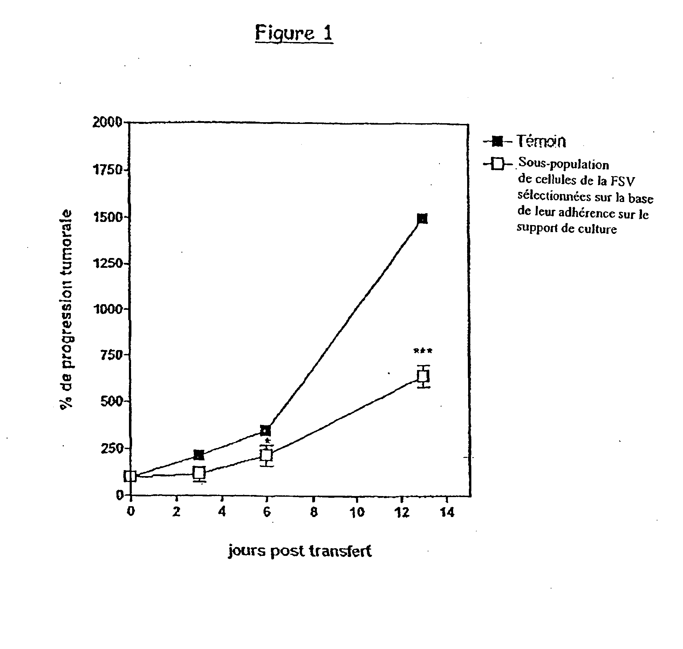

- Figure 1 : Détermination *in vivo* de la progression tumorale après injection intra-tumorale de la sous-population de cellules de la FSV sélectionnées sur la base de leur adhérence sur le support de culture (□) ou de PBS (■). Abscisses : nombre de jours après l'injection ; ordonnées : pourcentage de progression tumorale. * : p<0,05, *** : p<0,001.

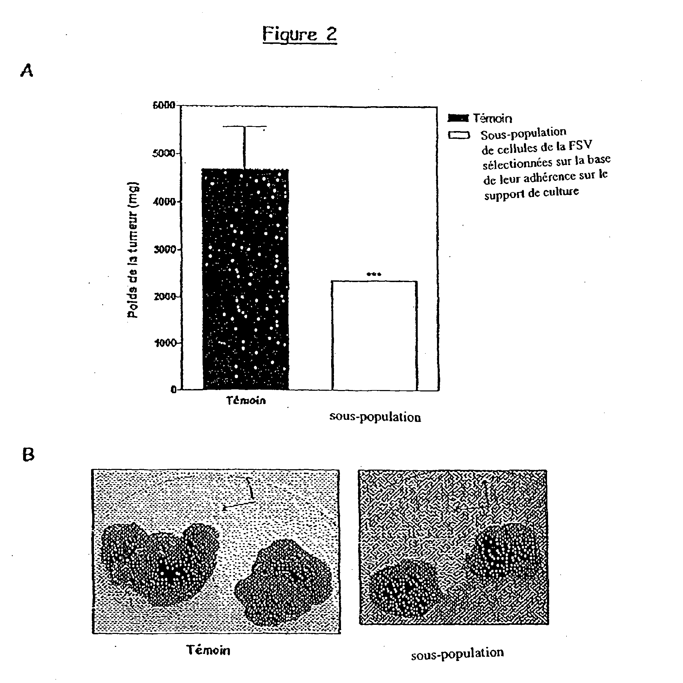

- Figure 2 : Diminution *in vivo* du poids de la tumeur pancréatique après injection intra-tumorale de la sous-population de cellules adhérentes. A : détermination du poids de la tumeur (mg) après injection intra-tumorale de cellules de la fraction stroma-vasculaire (blanc) ou de PBS (contrôle) (noir) ; *** : p<0,001. B : photographie de tumeurs prélevées après injection intra-tumorale de la sous-population de cellules adhérentes (droite) ou de PBS (gauche).

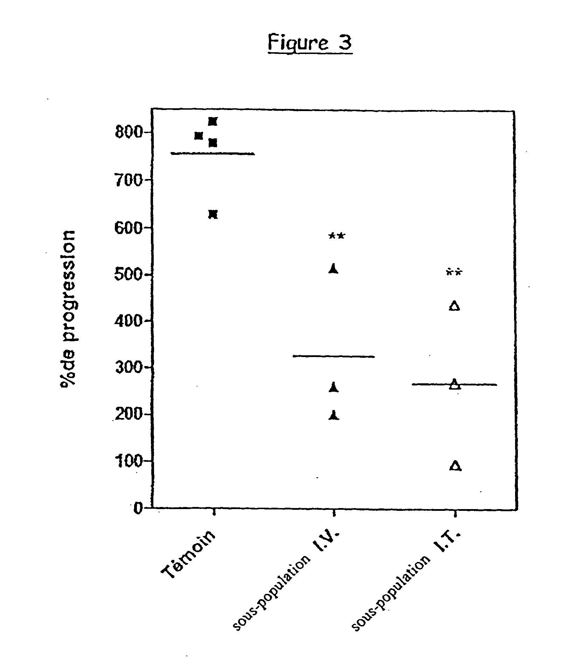

- Figure 3 : Détermination *in vivo* du pourcentage de progression tumorale après injection intra-tumorale (I.T. ; △) ou systémique (I.V. ; ▲) de la sous-population de cellules adhérentes, ou injection, soit intratumorale soit systémique, de PBS (Témoin ; ■). ** : p<0,01.

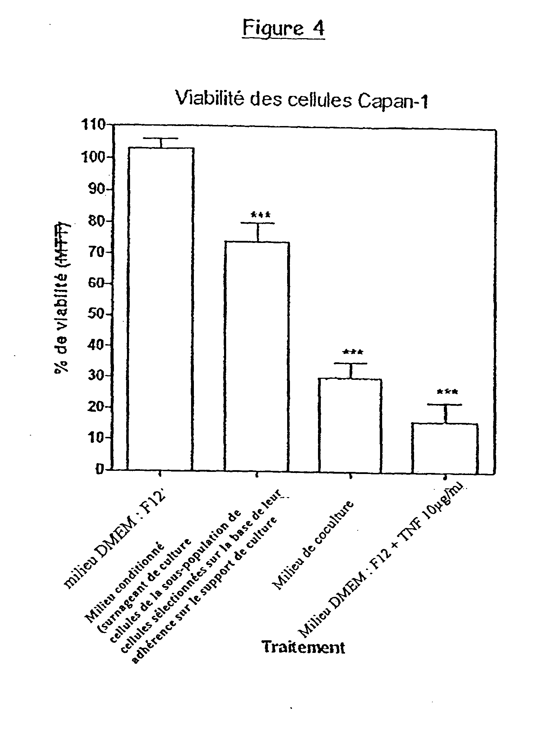

- Figure 4 : Détermination *in vitro* du pourcentage de viabilité des cellules Capan-1 traitées par du milieu DMEM:F12 OK, par du surnageant de culture de ladite sous-population de cellules (milieu conditionné), par du surnageant de co-culture de ladite sous-population de cellules et de cellules Capan-1 (milieu de co-culture) ou bien par du milieu DMEM : F12 supplémenté de 10 μg/ml de TNF-α. *** : p<0,001.

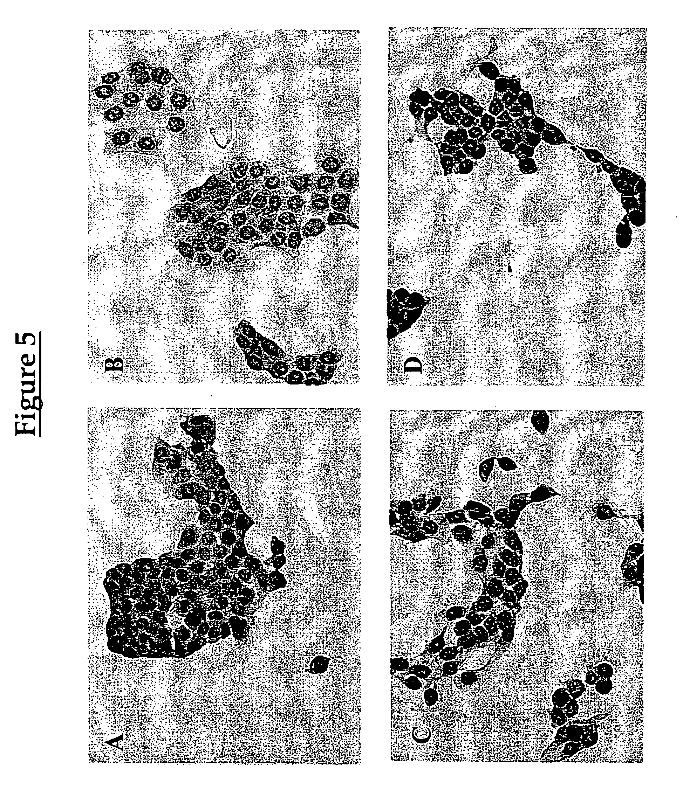

- Figure 5 : Détermination *in vitro* de l'apoptose des cellules Capan-1 induite par le surnageant de culture de cellules de la sous-population de cellules adhérentes et par le surnageant de co-culture des cellules de la sous-population de cellules adhérentes avec les cellules Capan-1. A : cellules Capan-1 mises en présence de milieu DMEM : F12 OK (témoin négatif). B : cellules Capan-1 traitées par le surnageant de culture de cellules de la sous-population de cellules adhérentes. C : cellules traitées par le surnageant de co-culture de cellules de la sous-population de cellules adhérentes avec les cellules Capan-1, selon un ratio Capan-1/cellules adhérentes de 5/1 ; D : cellules traitées par le surnageant de co-culture de cellules de la sous-population de cellules adhérentes avec les cellules Capan-1, selon un ratio Capan-1/cellules adhérentes de 1/1.

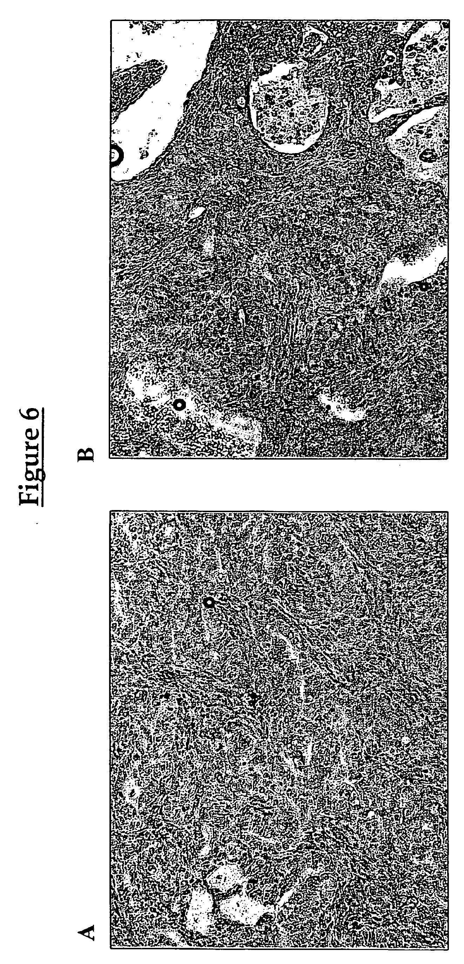

- Figure 6 : Détermination *in vivo* de l'apoptose des cellules cancéreuses après injection intra-tumorale de cellules de la sous-population de cellules adhérentes. Coupe de tumeur pancréatique témoin (A) ou après injection des cellules de la sous-population de cellules adhérentes (B).

Il doit être bien entendu, toutefois, que ces exemples sont donnés uniquement à titre d'illustration de l'objet de l'invention, dont ils ne constituent en aucune manière une limitation.

## EXEMPLE 1 : Matériels et Méthodes

### 1) Milieux utilisés

- Le milieu DMEM F12-OK comprend pour 500 ml de DMEM F 12 (référence Gibco 31330 038), 5 ml d'ASP (solution prête à l'emploi d'antibiotiques+antifongique : Amphotéricine 0,25 $\mu$g/ml, Streptomycine 100 $\mu$g/ml, Pénicilline G 100 $\mu$g/ml) (référence SIGMA A7292), 0,5 ml de biotine à 16 mM (0,016 mM final) (référence SIGMA B4639), 0,5 ml d'acide panthoténique à 18 mM (P5155 Sigma) (0,018 mM final), 0,5 ml d'acide ascorbique à 100 mM (A4034 Sigma) (100 $\mu$M final).
- Le tampon de digestion contient du DMEM F12-OK, 2 % de BSA (albumine sérique bovine) et 2 mg/ml de collagénase (référence SIGMA) à raison de 10 ml de milieu de digestion pour 3 g de tissu. Ce tampon est filtré à l'aide de filtres 0,2 $\mu$m (Acrodisc PF 0.8/0.2 $\mu$m, ref PALL6224187, VWR).
- Le tampon de lyse comprend 100 ml de solution A (2,08 g de tampon Tris pH 7,65 dans 100 ml $H_2O$ stérile) et 900 ml de solution B (8,3 g de $NH_4Cl$ dans 1000 ml $H_2O$ stérile).
- Le milieu RPMI complet est préparé à partir de milieu RPMI (Roswell Park Memorial Institute) 1640 (Gibco/Invitrogen (réf 21875034)) supplémenté de 10 % de sérum de veau nouveau-né (NCS ou « newborn calf serum » (Gibco 18010-159)), de 100 $\mu$/ml de pénicilline, 100 $\mu$/ml de streptomycine, et 0,25 $\mu$g/ml de fungizone (Invitrogen, 15240-096).
- La solution de PBS provient de chez Gibco (réf 14200-067).
- La solution de trypsine/EDTA provient de chez Gibco (réf 25300-054).

### 2) Obtention de la fraction stroma-vasculaire

Digestion

Le tissu adipeux provient de patients qui subissent une dermolipectomie ou une liposuccion :

*Pour une dermolipectomie :*

La quantité de tissu adipeux nécessaire est pesée dans une boîte stérile, puis l'ensemble du travail est fait sous hotte de culture. Le tissu subit une dissociation mécanique par un hachage très fin au moyen de ciseaux, et les fragments obtenus sont rincés avec du PBS.

Le tissu adipeux prélevé est disséqué sous microscope dans des boites stériles contenant du PBS, de manière à enlever toute trace de tissu musculaire, puis digéré à 37°C pendant 30 min, dans du milieu de digestion. La digestion est accélérée par une agitation manuelle toutes les 10 min.

*Pour une liposuccion :*

Le protocole est identique à l'exception de l'étape de dissociation mécanique du tissu à l'aide d'une paire de ciseaux, qui n'est pas nécessaire.

Purification de la fraction stroma-vasculaire (FSV ou SVF)

Après élimination des fragments non digérés par filtration (filtres de 25 $\mu$m, (PA 25/21, 25 $\mu$m, Tissage de Tissus Techniques, Sailly-Saillisel), les adipocytes matures sont séparés du culot contenant les cellules de la SVF par centrifugation (600 g, 10 min). Les cellules stroma-vasculaires ainsi isolées (culot) sont remises en suspension dans 2 ml de milieu DMEM:F12 + 10 % de SVN (Sérum de Veau Nouveau-né) et comptées (comptage manuel sur cellule hématimétrique ou compteur à particule Coulter) et les cellules remises en suspension dans le même milieu. Le même volume de tampon de lyse est ajouté et la suspension cellulaire est centrifugée 5 minutes à 1600 rpm (500 g). Le surnageant est éliminé et le culot repris dans du DMEM:F12 OK (de 500 $\mu$l à 1 ml en fonction de la taille du culot).

### 3) Culture cellulaire et obtention de la sous-population cellulaire

Après obtention de la fraction stroma vasculaire (FSV) brute, selon la méthode décrite ci-dessus, les cellules sont ensemencées dans des flacons de 25 cm$^2$ (Nunc col incliné et bouchon filtrant, ref 055422, Dominique Dutscher) à raison de 5x10$^5$ à 10$^6$ cellules par boîte et mises en culture dans le milieu DMEM : F12 OK. Les cellules sont rincées avec du PBS le lendemain de la mise en culture afin d'éliminer toutes les cellules mortes et/ou non adhérentes, puis elles sont cultivées pendant 4 à 7 jours dans du milieu DMEM:F12 OK + 10 % NCS.

Après confluence (soit après 4 à 7 jours de culture), les cellules sont rincées avec du PBS de manière à éliminer toute trace de milieu. Les cellules sont décollées à l'aide d'une solution de trypsine/EDTA puis comptées à l'aide d'un compteur de cellules (Coulter ZI). La suspension cellulaire est centrifugée 5 min à 1600 rpm (500 g), puis le culot est repris dans un volume adéquat de PBS, de manière à avoir une concentration de l'ordre de $10^6$ cellules/100 $\mu$l.

### 4) Lignée Capan-1 et modèle murin du cancer du pancréas humain

Un modèle murin du cancer du pancréas humain est mis au point comme indiqué ci-après. Ce modèle présente une tumeur ectopique au niveau sous-cutané.

#### - Lignée Capan-1

Les cellules Capan-1 sont dérivées de métastases hépatiques d'adénocarcinomes pancréatiques humains (ATCC HTB-79). Les cellules Capan-1 sont cultivées en routine dans le milieu de culture RPMI complet à 37°C et 5 % de $CO_2$, dans des flacons de cultures (référence BD Falcon T-175 353028), et sont repiquées lorsqu'elles atteignent 70 à 80 % de confluence.

#### - Souris Swiss nu/nu

Les souris femelles athymiques Swiss Nude (nu/nu) (Charles River) sont âgées de 6 à 8 semaines au moment de l'expérimentation. Après réception, les souris Swiss nu/nu sont tatouées à l'oreille pour identification ultérieure, puis acclimatées aux conditions d'élevage pendant 1 semaine en environnement A2 avant expérimentation (service de zootechnie de l'IFR31 à Toulouse), selon des cycles diurnes et nocturnes de 12 heures. Les souris Swiss nu/nu sont hébergées au nombre de 5 par cages sur portoir ventilé.

#### - Injection des cellules Capan- 1

*Préparation des suspensions cellulaires injectables*

Le milieu de culture de cellules Capan-1 est aspiré et les cellules rincées par 10 ml de PBS stérile. Après 5 minutes d'incubation à température ambiante, le PBS est aspiré, et les cellules sont dissociées à l'aide de 3 ml d'une solution de trypsine/EDTA, pendant 5 minutes à 37°C. Les cellules sont ensuite reprises dans 7 ml de milieu complet puis dissociées à la pipette après 10 cycles d'aspiration/refoulement, puis récoltées dans un tube de 50 ml stérile (Falcon Blue Max 50 ml 352070). Les cellules sont comptées à l'aide d'un compteur de cellules Z.I. Coulter. L'équivalent de $10^7$ cellules Capan-1 par souris à injecter est centrifugé pendant 5 min à 1400 rpm (200 g) en conditions stériles.

Le surnageant est éliminé, puis le culot est repris dans 25 ml de milieu complet puis dissocié à la pipette après 10 cycles d'aspiration/refoulement afin d'éliminer toute trace de trypsine. Après une centrifugation de 5 min à 1400 rpm (200 g), le surnageant est éliminé, et le culot est soumis à 3 cycles de lavages dans du milieu RPMI afin d'éliminer toute trace de sérum. Enfin, $10^7$ cellules Capan-1 sont reprises dans 100 $\mu$l de PBS stérile pour culture cellulaire.

*Implantation des tumeurs Capan-1*

Les souris, strictement manipulées sous hotte PSM (Poste de Sécurité Microbiologique), sont identifiées grâce à leur tatouage, pesées, puis anesthésiées par isofluorane 0,1 % (Aerrane, Baxter) pendant 5 minutes avant manipulation. Ce protocole d'anesthésie générale permet un confort dans la manipulation des individus et assure la reproductibilité des résultats, tout en prévenant des artefacts expérimentaux consécutifs au stress de l'animal. Après constatation de l'endormissement de l'animal, les cellules Capan-1 (100 $\mu$l) sont injectées en sous-cutané dans le flanc gauche de l'animal, à l'aide d'une seringue tuberculine 0,3 ml 29G sur 33 mm sans volume mort, à la vitesse de 1 ml/h. Le site de l'injection est désinfecté, et les souris reconditionnées dans des cages à litière propre. Le réveil des souris dans ces conditions d'expérimentation est constaté 5 à 7 minutes après anesthésie. Les signes vitaux des souris injectées sont analysés macroscopiquement 24 et 48 h suivant l'opération. Dans ces conditions, le taux de mortalité mesuré est de 0 %.

### 5) Injection des cellules de la sous-population de cellules adhérentes

L'injection de la sous-population de cellules adhérentes est réalisée 11 à 14 jours après implantation des tumeurs Capan-1 dans les souris athymiques Swiss nu/nu. Le volume moyen des tumeurs est alors de $250\pm18$ mm$^3$.

Comme indiqué précédemment, les souris, strictement manipulées sous hotte PSM, sont identifiées grâce à leur tatouage, pesées, puis anesthésiées par isofluorane 0,1 % pendant 5 minutes avant manipulation. Après constatation de l'endormissement de l'animal, les cellules ($5 \times 10^5$ dans 50 $\mu$l de PBS) sont injectées soit directement dans la tumeur, soit dans la veine caudale.

Pour la greffe intratumorale, les cellules sont injectées à l'aide d'une seringue tuberculine 0,3 ml 29G sur 33 mm sans volume mort, à la vitesse de 1 ml/h. Dans ce contexte, les cellules sont dans un environnement uniquement de cellules tumorales pancréatiques ; elles ne sont pas en contact avec le tissu pancréatique sain. Pour l'injection intraveineuse, les souris sont placées en chambre d'injection chauffée à 50°C pour faciliter la dilatation de la veine caudale, et les cellules sont injectées à l'aide d'un dispositif pour lymphographie sans volume mort de calibre 29G en PVC.

Dans les deux cas, 50 $\mu$l de PBS stérile sont injectés dans les souris contrôles soit au niveau intra-tumoral soit par voie systémique. Le site de l'injection est désinfecté, et les souris reconditionnées dans des cages à litière propre.

### 6) Analyse statistique

L'analyse statistique est réalisée à l'aide du logiciel Graphpad Instat V3.05. L'analyse de variance est réalisée par le test unilatéral ANOVA complété par un test de comparaisons multiples Student-Newman-Keuls. Une probabilité inférieure à 0.05 est considérée comme statistiquement significative.

### 7) Détection de l'apoptose par la technique TUNEL

L'apoptose des cellules cancéreuses est détectée sur des cultures de cellules Capan-1 ou sur des coupes de tumeurs pancréatiques provenant du modèle murin décrit dans l'Exemple 1.4) à l'aide de la technique TUNEL (terminal deoxynucleotidyl transferase mediated dUTP-biotin nick end labeling). Cette technique est basée sur l'incorporation de nucléotides marqués au niveau des extrémités 3'OH libres des fragments d'ADN générés lors de l'apoptose (Gavrieli et al., 1992, the Journal of Cell Biology, Vol. 119, n°3, pages 493-501). Elle est mise en oeuvre ici, à l'aide du kit ApopDETEK (EnzoDiagnostic, NY, USA) en suivant les recommandations du fabricant.

### <u>EXEMPLE 2</u> : Effet anti-tumoral *in vivo* des cellules dérivées du tissu adipeux

L'effet anti-tumoral des cellules de la fraction stroma-vasculaire du tissu adipeux extra-médullaire est évalué lorsque lesdites cellules sont injectées au niveau intra-tumoral ou par voie systémique chez des souris Swiss nu/nu développant une tumeur pancréatique dérivée des cellules Capan-1.

### 1) Injection intra-tumorale

Les conditions de préparation des souris, de l'injection des cellules Capan-1 et de l'injection de la sous-population de cellules adhérentes sont décrites dans l'Exemple I.

Après l'injection de la sous-population de cellules adhérentes, le poids des souris et la croissance des tumeurs sont mesurés et répertoriés tous les 2 jours et ce jusqu'à 14 jours suivant l'injection desdites cellules, la croissance tumorale étant mesurée *in situ* sur l'animal vivant. La mesure des longueurs (L) et largeurs (1) des tumeurs Capan-1 est réalisée à l'aide d'un pied à coulisse, selon le calcul suivant :

$$\text{Volume tumoral (mm}^3) = L^2 \text{ (mm) x l (mm) x 0,52}$$

Le pourcentage de progression tumorale est évalué 3, 6 et 13 jours après injection intra-tumorale desdites cellules ou de PBS (contrôle) selon la formule suivante :

$$\text{\% de progression tumoral au temps t} = [(\text{volume tumoral au temps t})/(\text{volume tumoral à } t_0)] \times 100 ; t_0 \text{ correspondant à l'injection de la sous-population de cellules adhérentes ou de PBS.}$$

L'expérimentation est stoppée 10 à 15 jours après transfert de la sous-population de cellules adhérentes (21 à 39 jours après implantation des tumeurs) en raison de la progression exponentielle des tumeurs témoins, de leur ulcération et du risque de décès élevé des souris portant ces tumeurs.

Outre la détermination de la progression tumorale au moyen de la mesure de la taille de la tumeur, le poids de la tumeur est également déterminé. Pour cela, les souris sont sacrifiées treize jours après injection intra-tumorale des cellules et les tumeurs sont prélevées, pesées et photographiées.

Les valeurs obtenues d'une part pour la progression tumorale et d'autre part pour la mesure du poids de la tumeur sont représentatives de 3 expériences indépendantes, correspondant à trois préparations différentes de la sous-population de cellules adhérentes, et 5 souris par groupe.

Les résultats du test d'inhibition de la progression tumorale sont illustrés dans la figure 1. Ils montrent une diminution drastique et significative de la taille des tumeurs Capan-1 de 46 % $\pm$ 13 %, 38 % $\pm$ 8 % et 57 % $\pm$ 14 % respectivement 3, 6 et 13 jours après l'injection des cellules de la sous-population. Ces données montrent un effet anti-tumoral se manifestant par la diminution drastique de la progression des tumeurs pancréatiques Capan-1 après transfert intra-tumoral de cellules. L'expérience n'a pu être poursuivie en raison de la croissance exponentielle et de l'ulcération des tumeurs témoins.

Les résultats relatifs à la mesure du poids et de la taille des tumeurs 13 jours après l'injection de la sous-population de cellules adhérentes sont illustrés dans la figure 2. Ils montrent une diminution de 50 % $\pm$ 0,1 % du poids des tumeurs Capan-1 après injection intra-tumorale desdites cellules. Ces résultats sont en accord avec ceux relatifs au pourcentage de progression tumorale, obtenus à partir de la mesure extrinsèque de la taille des tumeurs (figure 1) et confirment donc le rôle anti-tumoral des différentes cellules lorsque celles-ci sont injectées au niveau intra-tumoral.

**2) Injection systémique**

Parallèlement à ces études de diminution de la progression tumorale par une injection intra-tumorale de la sous-population de cellules adhérentes, les Inventeurs ont procédé à l'administration de ces cellules par voie sanguine, afin de déterminer si elles pouvaient migrer jusqu'au site de la tumeur et y exercer leur effet.

Les conditions de préparation des souris et d'injection des cellules Capan-1, de la sous-population de cellules adhérentes et du PBS sont décrites dans l'Exemple I. Plus précisément, les Inventeurs ont comparé l'effet des cellules de la sous-population administrées soit directement dans les tumeurs Capan-1 (injection intra-tumorale) soit dans la veine caudale (injection systémique). La taille des tumeurs est mesurée comme indiqué ci-dessus 3 jours après le transfert des cellules de la sous-population. Les valeurs obtenues sont représentatives de 2 expériences indépendantes correspondant à deux préparations de cellules différentes, et 3 ou 4 souris par groupe. Les résultats sont illustrés dans la figure 3. Ils montrent que l'amplitude de l'inhibition de la progression tumorale mesurée 3 jours après transfert des cellules de la FSV, lorsque celles-ci sont injectées dans la veine caudale des souris porteuses de tumeurs Capan-1, est comparable à celle observé lorsque ces cellules sont directement administrées dans la tumeur (-56 % $\pm$ 22 % vs -65 % $\pm$ 26 %).

Ces résultats sont en faveur d'un effet anti-tumoral *in vivo* des cellules de la sous-population lorsque celles-ci sont injectées par voie systémique.

L'ensemble des résultat présentés dans l'Exemple 2, montrant l'inhibition de la progression tumorale par les cellules de la sous-population injectées localement au niveau intra-tumoral ou bien administrées par voies systémique au niveau de la veine locale suggère fortement un rôle anti-tumoral des cellules de la fraction stroma-vasculaire du tissu adipeux blanc extra-médullaire.

En outre, ces résultats montrent le ciblage de la tumeur pancréatique par les cellules de la FSV sélectionnées sur la base de leur adhérence sur le support de culture lorsque celles-ci sont administrées à distance dans la circulation sanguine systémique.

**EXEMPLE 3 : Mesure de la viabilité cellulaire**

Les Inventeurs ont également mesuré la viabilité des cellules Capan-1 en présence de surnageant de cultures de la sous-population de cellules adhérentes *in vitro,* afin de confirmer et de valider les résultats obtenus *in vivo* (Exemple 2).

Les cellules Capan-1 sont ensemencées en sextuplés dans des boîtes de culture 96 puits à fond plat (Nunc 167008), à raison de 25000 cellules par puits dans un volume final de 100 $\mu$l, 48 h après, les cellules sont rincées puis traitées :

- par 100 $\mu$l de milieu RPMI sans sérum (témoin négatif, non représenté sur la figure 4) ;
- par 100 $\mu$l de milieu RPMI complet (témoin positif, non représenté sur la figure 4) ;
- par 100 $\mu$l de milieu DMEM : F12 supplémenté de 10 $\mu$g/ml de TNF-$\alpha$ (témoin positif d'inhibition de la viabilité) ;
- par 100 $\mu$l de milieu DMEM : F12 OK
- par 100 $\mu$l de surnageant de culture de cellules de la sous-population de cellules adhérentes ; ou
- par 100 $\mu$l de surnageant de co-culture de cellules de la sous-population de cellules adhérentes et de cellules Capan-1.

Le surnageant de culture de cellules de la sous-population de cellules adhérentes est obtenu après 48 heures de culture dans le milieu DMEM : F12 OK ; le surnageant de co-culture de cellules de la sous-population de cellules adhérentes et de cellules Capan-1 est obtenu après 48 heures d'une culture de cellules adhérentes et de cellules Capan-1 dans le milieu DMEM : F12OK, selon un rapport initial cellules Capan-1/cellules adhérentes de 5/1 ou de 1/1 (respectivement surnageant de co-culture 1 et surnageant de co-culture 2).

Deux jours (48 h) après, la mesure de la viabilité cellulaire est réalisée à l'aide du kit CellTiter 96® AQueous One Solution Cell Prolifération Assay (Promega G3582). Les valeurs obtenues sont représentatives de 3 expériences indépendantes correspondant à trois préparations de cellules de sous-population différentes.

Les résultats sont illustrés dans la figure 4. Ils montrent que le milieu conditionné par la sous-population de cellules adhérentes (surnageant de culture) est capable d'inhiber de façon significative la viabilité des cellules Capan-1. De plus, le surnageant de co-cultures de la sous-population de cellules adhérentes avec des cellules Capan-1 présente un effet inhibiteur sur la viabilité des cellules Capan-1 plus prononcé que le surnageant conditionné par la sous-population de cellules adhérentes seules, ce qui montre que ces cellules sont capables de réagir à la présence de cellules cancéreuses pancréatiques.

Ces résultats sont en faveur d'un effet anti-tumoral d'un milieu conditionné par les cellules de la fraction stroma-vasculaire du tissu adipeux blanc extra-médullaire, ledit effet pouvant être amplifié lorsque les cellules sont activées par des cellules cancéreuses telles que les cellules de la lignée Capan-1.

<u>EXEMPLE 4 :</u> **Induction de l'apoptose**

Les Inventeurs ont recherché si l'effet anti-tumoral des cellules de la fraction stroma-vasculaire du tissu adipeux blanc extra-médullaire et des surnageants de culture et de co-culture est dû à l'induction de l'apoptose des cellules cancéreuses.

**1) Induction *in vitro* de l'apoptose des cellules Capan-1**

Les cellules Capan-1 sont ensemencées en triplicate et cultivées sur Labteck 4 puits (Dutscher), à raison de 50 000 cellules par puits, dans un volume final de 500 $\mu$l, puis rincées et sont traitées par :

- 500 $\mu$l de milieu DMEM : F12OK ou de milieu RPMI 10 % ;
- 500 $\mu$l de surnageant de culture de cellules de la sous-population de cellules adhérentes ;
- 500 $\mu$l de surnageant de co-culture 1 ;
- 500 $\mu$l de surnageant de co-culture 2 ;

Le surnageant de culture de cellules de la sous-population de cellules adhérentes est obtenu comme indiqué dans l'Exemple 3.

24 heures après, l'apoptose est évaluée par la technique TUNEL comme indiqué dans l'Exemple 1.7).

Les résultats sont illustrés sur la Figure 5.

On observe un marquage nucléaire des cellules Capan-1 traitées par le surnageant de culture des cellules de la sous-population de cellules adhérentes (Figure 5B). Ce marquage n'est pas observé pour les cellules Capan-1 mises en présence de milieu DMEM : F12 OK ou de RPMI 10 % (Figure 5A). Pour les cellules Capan-1 traitées par le surnageant de co-culture 1, le marquage nucléaire est plus intense (Figure 5C) et l'intensité est encore augmentée par un traitement avec le surnageant de co-culture 2, pour lequel on observe en outre une condensation du cytoplasme (Figure 5D). Ces résultats montrent une augmentation quantitative de la fragmentation de l'ADN en présence de surnageant de co-culture, et donc une augmentation de l'apoptose des cellules Capan-1.

Ces résultats montrent donc que le surnageant de culture des cellules de la sous-population de cellules adhérentes, et le surnageant de co-culture des cellules de la sous-population de cellules adhérentes sont capables d'induire *in vitro* l'apoptose des cellules Capan-1.

**2) Induction *in vivo* de l'apoptose des cellules cancéreuses pancréatiques**

Les conditions de préparation des souris, de l'injection des cellules Capan-1 et de l'injection intra-tumorale de la sous-population de cellules adhérentes sont décrites dans l'Exemple I.

Cinq jours après l'injection des cellules de la sous-population de cellules adhérentes, des biopsies de tumeur pancréatique sont prélevées puis des coupes sont préparées. Pour le témoin négatif, des coupes de tumeurs sont préparées à partir de souris n'ayant pas reçu de cellules de la sous-population de cellules adhérentes. L'apoptose est détectée dans ces deux types de préparation à l'aide de la technique TUNEL comme indiqué dans l'Exemple 1.7).

Les résultats sont indiqués dans la Figue 6.

Aucun marquage n'est détecté dans les coupes de tumeurs provenant des souris témoin (Figure 6A). En revanche, on observe un marquage nucléaire pour de nombreuses cellules cancéreuses provenant de souris ayant subi l'injection de cellules de la sous-population de cellules adhérentes (Figure 6B) ce qui montre l'entrée en apoptose de ces cellules.

Ces résultats indiquent que les cellules de la sous-population des cellules adhérentes sont capables d'induire *in vivo* l'apoptose des cellules cancéreuses.

**Revendications**

1. Cellules isolées du tissu adipeux blanc extra-médullaire, sélectionnées dans le groupe constitué par la fraction stroma-vasculaire et une sous-population de ladite fraction stroma-vasculaire constituée par des cellules adhérentes, pour utilisation comme médicament anti-tumoral ayant pour effet le ralentissement ou l'inhibition de la croissance tumorale et l'induction de la mort des cellules cancéreuses.

2. Cellules isolées du tissu adipeux blanc extra-médullaire pour utilisation selon la revendication 1, **caractérisées en ce que** ladite sous-population de cellules adhérentes est susceptible d'être obtenue par un procédé comprenant :

   - l'obtention de la fraction stroma-vasculaire à partir du tissu adipeux blanc extra-médullaire ;
   - la purification de ladite fraction stroma-vasculaire ;
   - l'isolement de ladite sous-population de cellules à partir de ladite fraction purifiée par mise en culture primaire dans un milieu liquide convenable, sélection des cellules adhérentes sur un support de culture, récupération des cellules après confluence, dans un milieu convenable, centrifugation et récupération du culot.

3. Cellules isolées du tissu adipeux blanc extra-médullaire pour utilisation selon l'une quelconque des revendications 1 ou 2, **caractérisées en ce que** lesdites cellules sont associées à un véhicule phannaceutiquement acceptable.

4. Cellules isolées du tissu adipeux blanc extra-médullaire pour utilisation selon l'une quelconque des revendications 1 à 3, **caractérisées en ce que** lesdites cellules comprennent un gène hétérologue, dont le produit de traduction est une protéine d'intérêt thérapeutique, telle qu'une enzyme capable de métaboliser une pro-drogue en composé(s) actif(s) toxique(s) pour la cellule tumorale.

5. Cellules isolées du tissu adipeux blanc extra-médullaire pour utilisation selon les revendications 1 à 4, **caractérisées en ce que** ledit médicament anti-tumoral est constitué par le surnageant de culture de ladite sous-population de cellules adhérentes.

6. Cellules isolées du tissu adipeux blanc extra-médullaire pour utilisation selon la revendication 5, **caractérisées en ce que** ledit surnageant est obtenu à partir d'une co-culture de ladite sous-population de cellules avec des cellules cancéreuses ou avec des cellules d'une lignée cellulaire cancéreuse.

7. Cellules isolées du tissu adipeux blanc extra-médullaire pour utilisation selon l'une quelconque des revendications 1 à 6, **caractérisées en ce que** ledit cancer est un cancer solide ou un cancer liquide.

8. Cellules isolées du tissu adipeux blanc extra-médullaire pour utilisation selon la revendication 7, **caractérisées en ce que** ledit cancer est un cancer solide du tractus gastro-intestinal.

9. Cellules isolées du tissu adipeux blanc extra-médullaire pour utilisation selon la revendication 7, **caractérisées en ce que** ledit cancer est sélectionné dans le groupe constitué par le cancer du pancréas, le cancer gastrique et le cancer colo-rectal.

10. Agent anti-tumoral tel que défini aux revendications 1 à 6, à savoir sélectionné dans le groupe constitué par des cellules du tissu adipeux blanc extra-médullaire telles que définies à la revendication 1, le surnageant de culture desdites cellules ou le surnageant d'une co-culture desdites cellules avec des cellules cancéreuses ou des cellules d'une lignée cellulaire cancéreuse convenable, pour utilisation comme adjuvant en thérapie anticancéreuse.

11. Utilisation d'un agent anti-tumoral tel que défini aux revendications 1 à 6, à savoir sélectionné dans le groupe constitué par des cellules du tissu adipeux blanc extra-médullaire telles que définies à la revendication 1, le surnageant de culture desdites cellules ou le surnageant d'une co-culture desdites cellules avec des cellules cancéreuses ou des cellules d'une lignée cellulaire cancéreuse convenable, pour cribler *in vitro* d'autres médicaments anti-tumoraux, notamment aptes à agir en synergie avec lesdites cellules ou surnageant desdites cellules.

**Claims**

1. Cells isolated from extramedullary white adipose tissue selected from the group consisting of the stromal vascular fraction and a sub-population of the said stromal vascular fraction consisting of adherent cells, for use as an anti-tumour medicament having the effect of slowing down or inhibiting tumour growth and inducing the death of cancerous cells.

2. Cells isolated from extramedullary white adipose tissue for use according to claim 1, **characterized in that** the said sub-population of adherent cells can be obtained by a method comprising:

   - obtaining the stromal vascular fraction from extramedullary white adipose tissue;
   - purifying the said stromal vascular fraction;
   - isolating the said sub-population of cells from the said purified fraction by primary culturing in a suitable liquid medium, selection of adherent cells on a culture support, recovery of the cells after confluence, in a suitable medium, centrifugation and recovery of the pellet.

3. Cells isolated from extramedullary white adipose tissue for use according to any one of claims 1 or 2, **characterized in that** the said cells are associated with a pharmaceutically acceptable vehicle.

4. Cells isolated from extramedullary white adipose tissue for use according to any one of claims 1 to 3, **characterized in that** the said cells contain a heterologous gene, the translation product of which is a protein of therapeutic interest, such as an enzyme capable of metabolizing a prodrug into (an) active compound(s) which is/are toxic to the tumour cell.

5. Cells isolated from extramedullary white adipose tissue for use according to claims 1 to 4, **characterized in that** the said anti-tumour medicament consists of the culture supernatant of the said sub-population of adherent cells.

6. Cells isolated from extramedullary white adipose tissue for use according to claim 5, **characterized in that** the said supernatant is obtained from a co-culture of the said sub-population of cells with cancer cells or with cells of a cancer cell line.

7. Cells isolated from extramedullary white adipose tissue for use according to any one of claims 1 to 6, **characterized in that** the said cancer is a solid cancer or a liquid cancer.

8. Cells isolated from extramedullary white adipose tissue for use according to claim 7, **characterized in that** the said cancer is a solid cancer of the gastrointestinal tract.

9. Cells isolated from extramedullary white adipose tissue for use according to claim 7, **characterized in that** the said cancer is selected from the group consisting of cancer of the pancreas, gastric cancer and colorectal cancer.

10. Anti-tumour agent as defined in claims 1 to 6, that is to say selected from the group consisting of cells of extramedullary white adipose tissue as defined in claim 1, the culture supernatant of the said cells or the supernatant of a co-culture of the said cells with cancer cells or cells of a suitable cancer cell line, for use as an adjuvant in anticancer therapy.

11. Use of an anti-tumour agent as defined in claims 1 to 6, that is to say selected from the group consisting of cells of extramedullary white adipose tissue as defined in claim 1, the culture supernatant of the said cells or the supernatant of a co-culture of the said cells with cancer cells or cells of a suitable cancer cell line, for *in vitro* screening of other anti-tumour medicaments, in particular suitable for acting in synergy with the said cells or supernatant of the said cells.

**Patentansprüche**

1. Isolierte Zellen des extramedullären weißen Fettgewebes, ausgewählt aus der Gruppe, die aus der stroma-vaskullären Fraktion besteht, und einer Untergruppe der stroma-vaskullären Fraktion, die aus anhaftenden Zellen besteht, zur Verwendung als Antitumor-Medikament, das Verlangsamung oder Inhibierung des Tumorwachstums und Induktion des Absterbens der Krebszellen als Wirkung hat.

2. Isolierte Zellen des extramedullären weißen Fettgewebes zur Verwendung gemäß Anspruch 1, dadurch h gekennzeichnet, dass die Untergruppe von anhaftenden Zellen durch ein Verfahren erhalten werden kann, das umfasst:

   - Erhalten der stroma-vaskullären Fraktion aus dem extramedullären weißen Fettgewebe;
   - Reinigung der stroma-vaskullären Fraktion;
   - Isolierung der Untergruppe von Zellen aus der gereinigten Fraktion durch Durchführen einer Primärkultur in einem zweckmäßigen flüssigen Medium, Selektionieren der anhaftenden Zellen auf einem Kulturträger, Gewinnen der Zellen nach Konfluenz in einem zweckmäßigen Medium, Zentrifugation und Gewinnung des Rückstandes.

3. Isolierte Zellen des extramedullären weißen Fettgewebes zur Verwendung gemäß einem der Ansprüche 1 oder 2, **dadurch gekennzeichnet, dass** die Zellen mit einem pharmazeutisch verträgliche Vehikel assoziiert sind.

4. Isolierte Zellen des extramedullären weißen Fettgewebes, zur Verwendung gemäß einem der Ansprüche 1 bis 3, **dadurch gekennzeichnet, dass** die Zellen ein heterologes Gen umfassen, dessen Translationsprodukt ein Protein von therapeutischem Interesse ist, wie zum Beispiel ein Enzym, das in der Lage ist, ein Prodrug zu einer aktiven Verbindung (zu aktiven Verbindungen), die für die Tumorzelle toxisch ist (sind), zu metabolisieren.

5. Isolierte Zellen des extramedullären weißen Fettgewebes zur Verwendung gemäß den Ansprüchen 1 bis 4, **dadurch gekennzeichnet, dass** das Antitumor-Medikament vom Kulturüberstand der Untergruppe der anhaftenden Zellen gebildet wird.

6. Isolierte Zellen des extramedullären weißen Fettgewebes zur Verwendung gemäß Anspruch 5, **dadurch gekennzeichnet, dass** der Überstand aus einer Cokultur der Untergruppe von Zellen mit Krebszellen oder mit Zellen einer Krebszelllinie erhalten wird.

7. Isolierte Zellen des extramedullären weißen Fettgewebes zur Verwendung gemäß einem der Ansprüche 1 bis 6, **dadurch gekennzeichnet, dass** der Krebs ein solider Krebs oder ein flüssiger Krebs ist.

8. Isolierte Zellen des extramedullären weißen Fettgewebes zur Verwendung gemäß Anspruch 7, **dadurch gekennzeichnet, dass** der Krebs ein solider Krebs des Gastrointestinaltrakts ist.

9. Isolierte Zellen des extramedullären weißen Fettgewebes zur Verwendung gemäß Anspruch 7, **dadurch gekennzeichnet, dass** der Krebs aus der Gruppe, bestehend aus Krebs des Pankreas, Magenkrebs und kolorektalem Krebs, ausgewählt ist.

10. Antitumormittel, wie es in den Ansprüchen 1 bis 6 definiert ist, nämlich ausgewählt aus der Gruppe, bestehend aus Zellen des extramedullären weißen Fettgewebes, wie sie in Anspruch 1 definiert sind, dem Kulturüberstand der Zellen oder dem Überstand einer Cokultur der genannten Zellen mit Krebszellen oder Zellen einer zweckmäßigen Krebszelllinie, zur Verwendung als Adjuvans in der Antikrebstherapie.

11. Verwendung eines Antitumormittels, wie es in den Ansprüchen 1 bis 6 definiert ist, nämlich ausgewählt aus der Gruppe, bestehend aus Zellen des extramedullären weißen Fettgewebes, wie sie in Anspruch 1 definiert sind, dem Kulturüberstand der Zellen oder dem Kulturüberstand einer Cokultur der Zellen mit Krebszellen oder Zellen einer zweckmäßigen Krebszelllinie, um *in vitro* auf andere Antitumormedikamente, insbesondere solche, die in Synergie mit den Zellen oder dem Überstand der Zellen wirken, durchzumustern.

## Figure 1

## Figure 2

A

B

Témoin                              sous-population

## Figure 3

## Figure 4

Viabilité des cellules Capan-1

Figure 5

# Figure 6

A

B

EP 2 040 719 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 02055678 A
- US 20030082152 A
- EP 1077254 A
- US 6555374 B
- WO 0162901 A
- US 20030124721 A
- WO 03039481 A
- WO 2005025584 A

**Littérature non-brevet citée dans la description**

- **DIAZ-RUBIO.** *The Oncologist,* 2004, vol. 9, 282-294
- **SAFIOLEAS MC et al.** *Hepatogastroenterology,* 2004, vol. 51 (57), 862-868
- **JEMAL A. et al.** *CA Cancer J Clin,* 2002, vol. 52 (1), 23-47
- **ROSEWICZ S. et al.** *Lancet,* 1997, vol. 349 (9050), 485-489
- **JAFARI M. et al.** *Surg Oncol Clin N Am,* 2004, vol. 13 (4), 751-760
- **KULLKE MH et al.** *Curr Treat Options Oncol,* 2002, vol. 3 (6), 449-457
- **JAFARI M. et al.** *Surg Oncol Clin NAm,* 2004, vol. 13 (4), 751-760
- **MULVIHILL et al.** *Gene Therapy,* 2001, vol. 8, 308-315
- **HECHT et al.** *Clinical Cancer Research,* 2003, vol. 9, 555-561
- **SANGRO et al.** *Journal of Clinical Oncology,* 2004, vol. 22 (8), 1389-1397
- **MAZZOLINI et al.** *Journal of Clinical Oncology,* 2005, vol. 23 (5), 999-1010
- **SENZER N. et al.** *J Clin Oncol,* 2004, vol. 22 (4), 592-601
- **GÜNZBURG ; SALMONS.** *Acta Biochimica Polonica,* 2005, vol. 52 (3), 601-607
- **LÖHR et al.** *The Lancet,* 2001, vol. 357, 1591-1592
- **SAMEL et al.** *Cancer Gene Therapy,* 2006, vol. 13, 65-73
- **BJÖRNTORP et al.** *J. Lipid. Res.,* 1978, vol. 19, 316-24
- **PLANAT-BERNARD V. et al.** *Circulation,* 2004, vol. 109, 656-663
- **ZUK PA. et al.** *Mol. Biol. Cell,* 2002, vol. 13, 4279-95
- **ERICKSON GR et al.** *Biochem. Biophys. Res. Commun.,* 2002, vol. 290, 763-9
- **COUSIN B. et al.** *biochem. Biophys. Res. Commun.,* 2003, vol. 301, 1016-22
- **SAFFORD KM. et al.** *Biochem. Biophys. Res. Commun.,* 2002, vol. 294, 371-9
- **GAVRIELI et al.** *Journal of Cell Biology,* 1992, vol. 119 (3), 493-501